# EUROPEAN PATENT APPLICATION

(11) **EP 0 918 218 A2**
(43) Date of publication of application: **26.05.1999**
(21) Application number: 98121778.9
(22) Date of filing: 16.11.1998
(51) Int. Cl.: G01N 33/48

(54) **Method for immunological assay**

(30) Priority: 21.11.1997 JP 321110/97
(71) Applicant: DAIICHI PURE CHEMICALS CO. LTD., Chuo-ku Tokyo (JP)
(72) Inventor: Ukawa, Kenichi, c/o Daiichi Pure Chemicals Co., Naka-gun, Ibaraki (JP); Nakamura, Yasushi, c/o Daiichi Pure Chemicals Co., Naka-gun, Ibaraki (JP); Fukamachi, Isamu, c/o Daiichi Pure Chemicals Co., Naka-gun, Ibaraki (JP)
(74) Representative: Wächtershäuser, Günter, Prof. Dr.

(57) **Abstract**

An immunological assay method for an antigen or an antibody, which method makes use of a reaction between an immune complex resulting from a reaction between a target antigen Ag or a target antibody Ab1 to be measured and a corresponding antibody Ab1 or antigen Ag therefor and an antibody Ab2 that specifically reacts with the antibody that has formed the immune complex, to thereby measure antigen Ag or an antibody Ab1. The method of the present invention enables establishment of assay systems which have heretofore been impossible; i.e., immunonephelometry making use of a monoclonal antibody, latex agglutination making use of a monoclonal antibody, and hemagglutination making use of a monoclonal antibody; assay systems based on EIA or RIA, in which a single species of antibody is employed; and assay systems based on latex agglutination or hemagglutination using a trace amount of antibody.

## Description

### BACKGROUND OF THE INVENTION

### Field of the Invention

The present invention relates to a method for immunological assay making use of an antibody Ab2 that reacts specifically with an antibody that has formed an immune complex with an antigen Ag.

### Description of the Related Art

Conventionally, antigens and specific antibodies have been measured by immunological assay.

As assay methods for antigens, there have been known radioimmunoassay (RIA), enzyme immunoassay (EIA), immunonephelometry, latex agglutination, and hemagglutination. Of these methods, radioimmunoassay (RIA) and enzyme immunoassay (EIA) employ either an antibody labeled with a radioactive substance or an enzyme to perform an assay for an antigen based on its radioactive activity or enzyme activity after an antigen-antibody reaction. According to immunonephelometry, the amount of an antigen is measured based on the change in absorbance of turbidity that occurs as a result of antigen-antibody reaction. In latex agglutination and hemagglutination, the amount of an antigen is measured according to the extent of agglutination caused by a reaction between an antigen and antibody-sensitized latex particles or between an antigen and antibody-sensitized erythrocytes.

As assay methods for antibodies, there have been known RIA and EIA, in which an antibody that has specifically been bound, through antigen-antibody reaction, to an antigen immobilized onto a polystyrene plate or onto a polystyrene ball is measured by use of a secondary antibody labeled with a radioactive substance or an enzyme; and latex agglutination and hemagglutination, in which an amount of an antibody is measured according to the extent of an agglutination caused by a reaction between an antibody and antigen-sensitized latex particles or between an antibody and antigen-sensitized erythrocytes.

However, conventional methods for measuring an antigen have a drawback in that applicable assay methods are restricted by concentration and properties of the antigen, the antibody to be used, etc., and those for measuring an antibody also have a drawback in that applicable assay methods are restricted by concentration of the antibody, etc.

In methods for measuring an antigen by use of a single monoclonal antibody, monofunctional antigens have not been able to be assayed by immunonephelometry, latex agglutination, or hemagglutination due to characteristics of a monoclonal antibody. For measuring a hapten, immunonephelometry, latex agglutination, and hemagglutination are impossible to perform, since the characteristics of the hapten's antigenecity impose extreme difficulties in preparation of several types of antibodies; and in most cases, EIA and RIA are also restricted to those which use a competitive assay. Mention may be given of a sandwich technique as another assay method, but this technique is intricate as it requires two or more different antibodies.

In assay methods for an antibody, due to lack of sensitivity, latex agglutination and hemagglutination are not suitable for measuring an antibody which is present in a very small amount.

In view of the foregoing, the inventors of the present invention have carried out earnest studies and have found that an antibody that has formed an immune complex through reaction with an antigen undergoes a partial change in its structure, and that use of an antibody that recognizes the changed portion can solve the above-mentioned problems, leading to completion of the invention.

### SUMMARY OF THE INVENTION

Accordingly, an object of the present invention is to provide a novel immunological assay method that can be widely used in a variety of assay formats without imposing any limitation on the object to which the method is applied, irrespective of concentration and properties of an antigen or antibody, or the type of the antibody or the like to be used.

The present invention provides an immunological assay method for an antigen Ag or an antibody Ab1, which method makes use of a reaction between an immune complex resulting from a reaction between a target antigen Ag or a target antibody Ab1 to be measured and a corresponding antibody Ab1 or antigen Ag therefor and an antibody Ab2 that specifically reacts with the antibody that has formed the immune complex.

### BRIEF DESCRIPTION OF THE DRAWINGS

FIG. 1 is a graph showing the specificity, as determined by ELISA, of the polyclonal antibody obtained in Production Example 1.
FIG. 2 is a graph showing the specificity, as determined by ELISA, of monoclonal antibody 54201 obtained in Production Example 2.
FIG. 3 is a graph showing the specificity, as determined by Western blotting, of monoclonal antibody 54201 obtained in Production Example 2.
FIG. 4 is a graph showing the relationship between the concentration of an antigen (ATIII) and absorbance, as found in the case in which monoclonal antibody 54201 was used in ELISA.
FIG. 5 is a graph showing the relationship between the concentration of an antigen (ATIII) and absorbance, as found in the case in which latex sensitized with monoclonal antibody 54201 was used in a latex agglutination reaction system.
FIG. 6 is a graph showing the relationship between the concentration of an antigen (ATIII) and absorbance, as found in the case in which monoclonal antibody 54201 was used in a latex agglutination reaction system.

### BEST MODE FOR CARRYING OUT THE INVENTION

The antibody Ab2 which is used in the present invention may be polyclonal or monoclonal, and may respectively be prepared as described below.

Polyclonal antibodies may be prepared by the steps of, for example, immunizing an animal through administration of immune complexes that have been formed from a reaction between antigens Ag and corresponding antibodies Ab1, collecting an antiserum, and removing from the thus-collected antiserum antibodies for the antigens Ag and antibodies for unreacted antibodies Ab1.

Preferably, the immune complexes that serve as immunogens are treated with a cross-linking agent such as glutaraldehyde. Animals which are used for immunization are generally rabbits, goats, and chickens. Customary methods may be used for immunization. For example, an immunogen suspended in a typical buffer or saline, or mixed with an adjuvant such as complete Freund's adjuvant, is administered to an animal subcutaneously, intracutaneously, or peritoneally. If necessary, this procedure is performed repeatedly. The dose of the immune complex is suitably determined in accordance with the route of administration, animal species, etc. Under normal circumstances, the dose is approximately between 10 µg and 1 mg per administration. When antibodies against immune complexes are clearly confirmed in the serum of the immunized animal, blood is collected from, for example, the heart by use of an injector, to thereby obtain serum.

Since the thus-collected serum contains antibodies other than the polyclonal antibodies Ab2 of interest, these antibodies must be removed by immunoadsorption or like techniques. For example, there may be performed removal of antibodies against antigens Ag and antibodies against unreacted antibodies Ab1 by causing the antiserum to pass through an antigen Ag-binding affinity column and an antibody Ab1-binding affinity column provides the target polyclonal antibodies Ab2.

Monoclonal antibodies may be prepared by the steps of, for example, immunizing an animal through administration of immune complexes that have been formed from a reaction between antigens Ag and corresponding antibodies Ab1; causing cell fusion between the resultant antibody-producing cells and myeloma cells; choosing, from the obtained hybridomas, hybridomas capable of producing antibodies which specifically react with the antibodies that form the immunocomplexes; and growing the thus-collected chosen hybridomas in the abdominal cavity of an animal or in a culture liquid; and subsequently collecting the monoclonal antibodies Ab2 from the ascites or culture liquid.

Animals which are used for immunization are not particularly limited, and generally, mice and rats are used. Immunization may be performed through customary methods. Antibody-producing cells which are used in the subsequent cell fusion process are preferably cells from the spleen that is removed three to four days after the final immunization. The myeloma cells which serve as the parent cells to be fused with the aforementioned antibody-producing cells, there may be used a variety of known cell strains which have been established, such as NS1(P3/NSI/1-Ag4-1) [Eur. J. Immunol. 6:511-519 (1976)], SP2/0-Ag14 [Nature 276:269 (1978)], P3X63-Ag8.653 [J. Immunol. 123:1548 (1979], and P3X63-Ag8U.1 [Curr. Top. Microbiol. Immunol. 81:1 (1978)], all from mice; and Y3-Ag1.2.3. [Nature 277: 131-133 (1979)] and YB2/0(YB2/3HL/P2.G11.16Ag.20) [Methods Enzymol. 73B:1 (1981)] from rats. Cell fusion may be performed through use of polyethylene glycol (PEG), Sendai virus (HVJ), etc., which are routinely used, in a conventional manner; for example, immunocytes are used in an amount of approximately 1-10 times that of myeloma cells, and polyethylene glycol having an average molecular weight of 1000-6000 is used at a concentration of 30-60%; and the polyethylene glycol is added dropwise to a pellet of a mixture of the immunocytes and myeloma cells.

Screening of hybridoma cells is carried out by use of a common selection medium, for example, a HAT medium (a medium containing hypoxanthine, aminopterin, and thymidine). The hybridomas obtained through culturing in a HAT medium are subjected to routine limiting dilution method to thereby screen for strains that produce the target antibody for establishing monoclonal antibodies. In order to obtain the target antibody-producing cell strains, there may be used ELISA, RIA, etc., which allow for selection of antibody-producing hybridomas that specifically react with antibodies that have formed immune complexes. Monoclonal antibodies Ab2 are prepared from the thus-obtained target-antibody-producing hybridomas in a customary method. Briefly, monoclonal antibodies may be separated from a culture supernatent obtained by culturing the hybridomas by a customary method. Alternatively, they may be recovered as an ascites after the hybridomas are administered to a mammal which is compatible with the hybridomas.

The immunological assay of the present invention makes use of a reaction between the thus-obtained antibody Ab2 and the immunocomplex obtained from the reaction between a to-be-assayed antigen Ag or to-be-assayed antibody Ab1 and the corresponding antibody Ab1 or antigen Ag. This method is applicable to arbitrary conventional immunological assay.

The assay formats are not particularly limited. For example, EIA, RIA, immunonephelometry, latex agglutination, and hemagglutination.

More specifically, when antigens or antibodies are assayed by EIA or RIA, immobilzed antibody Ab1 or antigen Ag is added to a sample containing a to-be-assayed antigen Ag or to-be-assayed antibody Ab1, to thereby form an immune complex, which is then caused to react with antibody Ab2 which has been labeled with an enzyme, a radioactive substance, a fluorophor, or a chemical luminescent substance. Subsequently, the activity of the labeling substance in the solid phase is measured to thereby quantitatively determine the antigen Ag or the antibody Ab.

When antigens or antibodies are assayed by immunonephelometry, antibody Ab1 or antigen Ag is added to a sample containing a to-be-assayed antigen Ag or to-be-assayed antibody Ab1, to thereby form an immune complex. An antibody Ab2 is then added thereto, and based on the absorbance changes caused by the reaction, the antigen Ag or antibody Ab1 can be quantitatively determined.

When antigens or antibodies are assayed by latex agglutination or hemagglutination, the following two formats may be applicable: a format in which a carrier (latex particles or blood cells) is directly sensitized with antibody Ab2; and a format in which an antibody is used in the form of a liquid phase antibody. According to the former format, antibody Ab1 or antigen Ag is added to a sample containing a to-be-assayed antigen Ag or a to-be-assayed antibody Ab1, to thereby form an immune complex. Subsequently, antibody Ab2-sensitized carrier is then added thereto, and based on the absorbance changes or extent of agglutination of the materials used as the carrier, the antigen Ag or antibody Ab1 can be quantitatively determined. On the other hand, according to the latter format, a carrier sensitized with antibody Ab1 or antigen Ag is added to a sample containing a to-be-assayed antigen Ag or a to-be-assayed antibody Ab1, to thereby form an immune complex. Subsequently, antibody Ab2 is added thereto, and based on the absorbance changes resulting from the reaction or the extent of agglutination of the materials used as the carrier, the antigen Ag or antibody Ab1 can be quantitatively determined.

Of the above-described assay formats, the method of the present invention is particularly useful in antigen assays by EIA, RIA, immunonephelometry, latex agglutination, and hemagglutination making use of monoclonal antibodies; and in antibody assays by latex agglutination and hemagglutination.

### Examples

The present invention will next be described in detail by way of examples, which should not be construed as limiting the invention thereto.

### Production Example 1 (Preparation of Polyclonal Antibody)

Anti-thrombin III (ATIII) and its monoclonal antibody (Mab) 13208 (produced by hybridoma 13208: FERM BP-6522) were incubated at room temperature for seven hours. Subsequently, the resultant immune complexes were purified with a heparin-conjugated Sepharose 4B column and then with a protein A-conjugated Sepharose 4B column. The immune complexes were treated with 0.125% glutaraldehyde at room temperature for two hours, and the salts were removed with a PD-10 column (Pharmacia). The immune complexes thus treated were used as immunogens. The immunogens were emulsified with complete Freund's adjuvant at a ratio of 1:1, and 2 ml of the emulsion (0.05 mg/ml) was subcutaneously injected into a rabbit (Japanese white rabbit) at two-week intervals, seven times in total. Blood was collected through a rabbit vein, to thereby obtain antiserum. The thus-obtained antiserum was passed through both a Sepharose 4B column conjugated with Mab13208 and a Sepharose 4B column conjugated with ATIII. The polyclonal antibody thus obtained was used for the present invention.

### Example 1 (Specificity of the Polyclonal Antibody)

ATIII, mouse IgG (Mab13208), or their immune complexes were respectively adjusted to have an absorbance of 1 mOD/ml at 280 nm by use of 20 mM phosphate-buffered saline (PBS) (pH 7.2), and the resultant solutions were respectively aliquoted to an ELISA 96-well plate (Nunk) (50 µl/well). The plate was incubated at 4°C overnight. After 3 washings with PBS containing 0.05% Tween 20 (T-PBS), the plate was blocked for 2 h by addition of 300 µl T-PBS to each well. The polyclonal antibody described above in Production Example 1 was diluted to 100 mOD/ml at 280 nm, and after the blocking solution was removed, 50 µl of the diluted polyclonal antibody was added to each well and incubated at room temperature for 2 h. Then the plate was washed three times with T-PBS, followed by incubation at room temperature for 2 h in 50 µl (per well) of a peroxidase-conjugated anti-rabbit IgG solution diluted 5000-fold with T-PBS. After three washings with T-PBS, 50 µl of a peroxidase substrate solution was added to each well and incubated for 15 min. Then 50 µl of 1.5 N sulfuric acid was added to each well, and absorbance of each sample was measured at 492 nm.

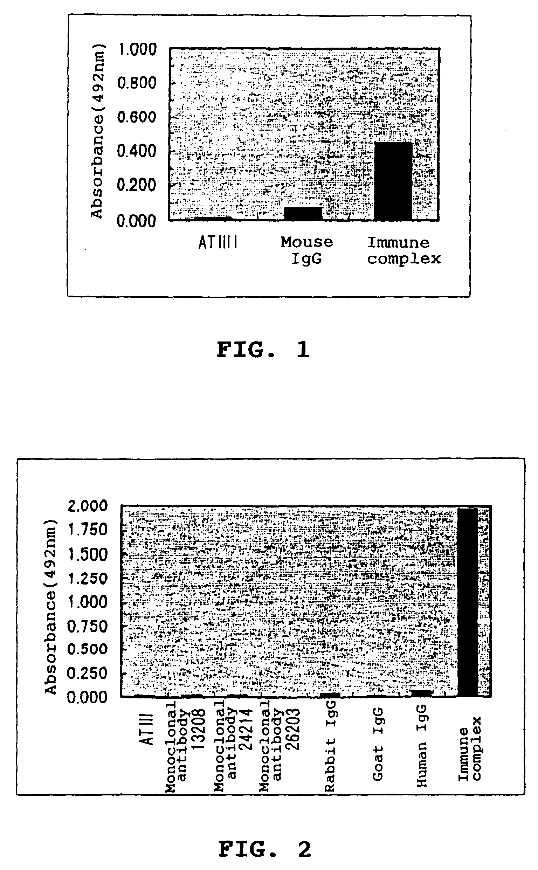

The results are shown in FIG. 1, demonstrating that the polyclonal antibody obtained in Production Example 1 reacted specifically with the immune complexes.

### Production Example 2 (Preparation of Monoclonal Antibody)

### (1) Preparation of Hybridoma

The immune complexes, prepared as described above in connection with the preparation of polyclonal antibody, were treated at room temperature for 2 h with 5 mM l-ethyl-3-(3-dimethyl aminopropyl)carbodiimide hydrochloride, desalted by being passed through a PD-10 column, and used as immunogens. The antigens emulsified with complete Freund's adjuvant (1:1) were injected subcutaneously to the back of a female Wistar rat at 7 weeks of age. The injection was repeated 9 times and then, 3 days after the final injection, the spleen was removed. The spleen cells and SP2/0-Ag14 myeloma cells were mixed at a ratio of 3:1, and were subjected to cell fusion in the presence of 50% polyethylene glycol 1540 (Wako Pure Chemical Industries, Ltd.). The fused cells were suspended in HAT medium at a concentration equivalent to 2 x 10⁶ spleen cells per ml, and the cell suspension was plated onto a 24-well culture plate (Corning) (2 ml/well). The cells were then cultured at 37°C in a 5% CO₂-incubator. Supernatants of cultures showing cell proliferation were further tested by ELISA for immune reactivity against ATIII, MAb13208, and their immune complexes. Hybridoma cells producing antibodies capable of reacting specifically to the immune complexes were further subjected to cellular cloning by limiting dilution analysis, to thereby obtain an Mab-producing hybridoma 54201 (FERM BP-6521).

### (2) Preparation of Monoclonal Antibody

Hybridoma 54201 cells obtained in the above (1) were suspended at 1 x 10⁵ cells per ml in RPMI medium with 15% fetal calf serum, and 50 ml of the suspension was added to a 300 ml-culture bottle (Corning) for incubation at 37°C for 4 days in a 5%-CO₂ incubator. The cultured supernatants obtained by centrifugation were passed through a Protein G-conjugated column (Pharmacia) to retain the monoclonal antibody. The trapped antibody Mab54201 was then eluted with a 0.1 M glycine-hydrochloride buffer (pH 3.0).

### Example 2 (Specificity of the Monoclonal Antibody)

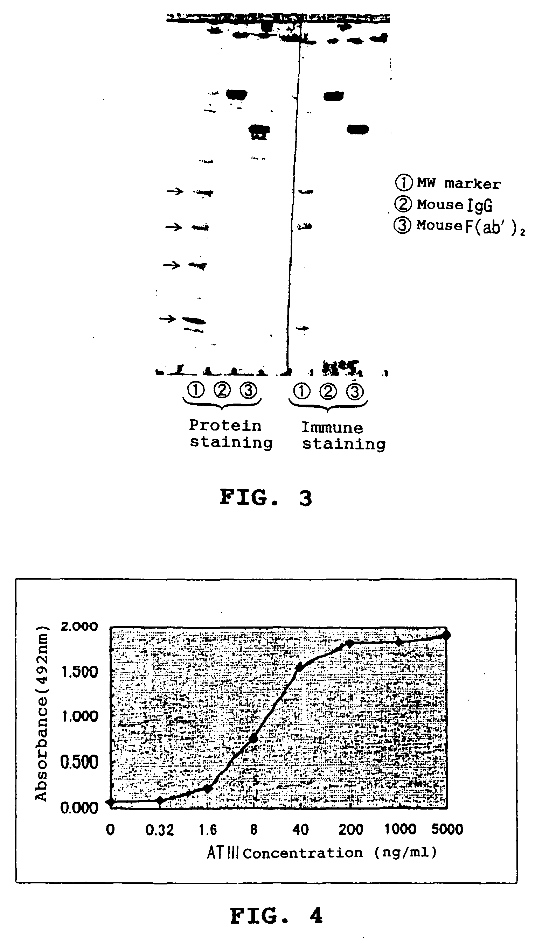

(1) ELISA Method
   Each of ATIII, mouse IgG (Mab13208, 24214, and 26203), rabbit IgG, human IgG, goat IgG, and the immune complexes formed between ATIII and Mab13208 was adjusted to an absorbance of 1 mOD/ml at 280 nm with 20 mM phosphate-buffered saline (PBS) (pH 7.2); each sample was respectively aliquoted to 96-well ELISA plates (Nunk; 50 µl/well); and the plates were incubated at 4°C overnight, washed 3 times with T-PBS, and blocked for 2 hours with 300 µl (per well) of TBS containing 0.1% skim milk. After the blocking solution was discarded, 50 µl of the hybridoma supernatant containing Mab54201 that had been obtained in the above Production Example 2 (2) was added to each well, and the plates were incubated at room temperature for 2 h. Then, after the plate was washed with T-PBS 3 times, 50 µl of a peroxidase-conjugated rat IgG (BioSource) solution diluted 5000-fold with T-PBS was added to each well, and the plates were incubated at room temperature for 2 h. The plates were washed with T-PBS 3 times, and then 50 µl of a peroxidase substrate solution was added to each well. After 15 min, the reaction was stopped by addition of 50 µl of 1.5 N sulfuric acid to each well, and absorbance of each sample was measured at 492 nm.
   The results are shown in FIG. 2, demonstrating that Mab54201 was able to react specifically with the immune complexes.
(2) Western Blotting Method
   IgG and F(ab')₂ of Mab13208 were subjected to SDS-polyacrylamide gel electrophoresis, and electrically transferred onto a PVDF membrane filter (Millipore). The filter was blocked for 2 h with T-PBS containing 1% skim milk, and was caused to react with a first antibody of Mab54201 and with a second antibody of peroxidase-conjugated anti-rat IgG. After the PVDF membrane was washed with T-PBS, the color reaction was carried out by addition of a substrate of diaminobenzidine. As shown in FIG. 3, Mab54201 was able to react with the IgG and F(ab')₂, indicating that the epitope for Mab54201 was present within F(ab')₂.
(3) Taken together, the above results obtained by ELISA and Western blotting analysis indicate that Mab54201 reacted with neither ATIII nor intact IgG (Mab13208), but that it was able to react with mouse IgG denatured with SDS or mouse IgG(Mab13208) that had formed immune complexes.

### Example 3 (Application to Antigen Measurement System by ELISA)

Mab13208 (0.139 µg/ml) for ATIII was aliquoted to a 96-well ELISA plate (Nunk)(50 µl/well), and the plate was incubated at 4°C overnight, then washed 3 times with T-PBS, and blocked for 2 h with 300 µl per well of TBS containing 0.1% skim milk. After the blocking solution was removed, various concentrations of diluted standard solutions of ATIII (0, 1.6, 8, 40, and 200 ng/ml) were added to wells (50 µl/well), and the plate was incubated at room temperature for 2 h. Then, after the plate was washed with T-PBS 3 times, 50 µl of the hybridoma supernatant for Mab54201 that had been obtained in the above Production Example 2 (2) was added to each well, and incubated at room temperature for 2 h. After the plate was washed with T-PBS 3 times, 50 µl of a peroxidase-conjugated anti-rat IgG (BioSource) solution diluted 5000-fold with T-PBS was added to each well. The plate was then incubated at room temperature for 2 h, washed with T-PBS 3 times, and 50 µl of a peroxidase substrate solution was added to each well. After 15 min, the reaction was stopped by addition of 50 µl of 1.5 N sulfuric acid to each well, and absorbance of each sample at 492 nm was measured.

The obtained results are shown in FIG. 4, indicating that the absorbance was dependent on concentrations of ATIII. Example 4 (Application to Antigen Measurement System by Latex Agglutination)

### (1) Mab54201-Sensitized Latex System

Mab54201(1 mg/ml) was dialyzed against a 50 mM glycine buffer (pH 8.4), and a 2% solution of 0.15 µm latex (Sekisui) was also prepared with the same buffer. By mixing of an equal volume of each solution, latex particles were sensitized at 4°C for 2 h, blocked with 50 mM glycine buffer (pH 8.4) containing 0.5% bovine serum albumin (BSA), and subjected to aging in a 50 mM glycine buffer (pH 8.4) containing 0.1% BSA and 0.005% Tween 80. Then the latex particles were suspended in a 50 mM glycine buffer (pH 8.4) containing 0.1% BSA and 10% glycerol, and the preparation thus obtained was called Mab54201-sensitized latex. To 600 µl of the sensitized latex solution (0.05%), Mab13208 (30 µl/ml) against ATIII was added, and subsequently 10 µl of ATIII at one of various concentrations was further added. Absorbance of each sample was measured at 600 nm with a spectrophotometer.

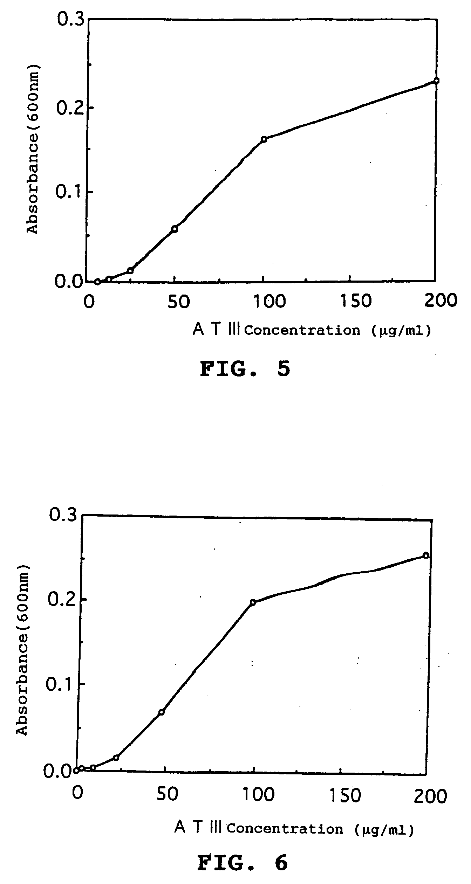

The results, as shown in FIG. 5, indicate that the absorbance increased in proportion to the ATIII concentration, demonstrating that ATIII can be assayed by use of the sensitized latex.

### (2) Mab13208-Sensitized Latex System

Mab13208 (1.68 mg/ml) against ATIII was dialyzed against a 50 mM glycine buffer (pH 8.4), and a 2% solution of 0.15 µm latex (Sekisui) was also prepared with the same buffer. By mixing of an equal volume of each solution, latex particles were sensitized at 4°C for 2 h, blocked with 50 mM glycine buffer (pH 8.4) containing 0.5% bovine serum albumin (BSA), and subjected to aging in a 50 mM glycine buffer (pH 8.4) containing 0.1% BSA and 0.005% Tween 80. Then the latex particles were suspended in a 50 mM glycine buffer (pH 8.4) containing 0.1% BSA and 10% glycerol, and the preparation thus obtained was called Mab54201-sensitized latex. To 600 µl of the sensitized latex solution (0.05%), Mab54201 (10 µl/ml) was added, and subsequently 10 µl of ATIII at one of various concentrations was further added. Absorbance of each sample was measured at 600 nm with a spectrophotometer.

The results, as shown in FIG. 6, indicate that the absorbance increased in proportion to the ATIII concentration, demonstrating that ATIII can be measured by use of the sensitized latex.

As described above, according to the present invention, an immunoassay is performed by making use of an antibody Ab2 that reacts specifically with an antibody that has formed an immune complex with an antigen Ag. This enables establishment of assay systems which have heretofore been impossible; i.e., immunonephelometry making use of a monoclonal antibody, latex agglutination making use of a monoclonal antibody, and hemagglutination making use of a monoclonal antibody; assay systems based on EIA or RIA, in which a single species of antibody is employed; and assay systems based on latex agglutination or hemagglutination using a trace amount of antibody.

## Claims

1. An immunological assay method for an antigen or an antibody, which method makes use of a reaction between an immune complex resulting from a reaction between a target antigen Ag or a target antibody Ab1 to be measured and a corresponding antibody Ab1 or antigen Ag therefor and an antibody Ab2 that specifically reacts with the antibody that has formed the immune complex, to thereby measure the antigen Ag or an antibody Ab1

2. An immunological assay method according to Claim 1, wherein the immune complex is formed by addition of immobilzed antibody Ab1 or antigen Ag to a sample containing antigen Ag to be measured or antibody Ab1 to be measured; the thus-formed immune complex is caused to react with antibody Ab2 which specifically reacts with the antibody that has formed the immune complex, the antibody Ab2 being labeled with an enzyme, a radioactive substance, a fluorophor, or a chemical luminescent substance; and subsequently, the activity of the labeling substance in the solid phase is measured to thereby quantitating the antigen Ag or the antibody Ab1.

3. An immunological assay method according to Claim 1, wherein antibody Ab1 or antigen Ag is added to a sample containing antigen Ag to be measured or an antibody Ab1 to be measured to thereby form the immune complex; antibody Ab2 which specifically reacts with the antibody that has formed the immune complex is added to the immune complex so as to cause a reaction; and subsequently the antigen Ag or the antibody Ab1 is quantitatively determined based on induced changes in absorbance.

4. An immunological assay method according to Claim 1, wherein antibody Ab1 or antigen Ag is added to a sample containing antigen Ag to be measured or an antibody Ab1 to be measured to thereby form an immune complex; a carrier sensitized with antibody Ab2 which specifically reacts with the antibody that has formed the immune complex is added to the immune complex so as to cause a reaction; and subsequently the antigen Ag or the antibody Ab is quantitatively determined based on induced changes in absorbance.

5. An immunological assay method according to Claim 1, wherein a carrier sensitized with antibody Ab1 or with antigen Ag is added to a sample containing antigen Ag to be measured or an antibody Ab1 to be measured to thereby form an immune complex; antibody Ab2 which specifically reacts with the antibody that has formed the immune complex is added to the immune complex so as to cause a reaction; and subsequently the antigen Ag or the antibody Ab is quantitatively determined based on induced changes in absorbance or the extent of aggulutination of the carrier.
